# DEMANDE DE BREVET EUROPEEN

(11) **EP 1 634 620 A1**
(43) Date de publication de la demande: **15.03.2006**
(21) Numéro de dépôt: 05300732.4
(22) Date de dépôt: 08.09.2005
(51) Int. Cl.: A61Q 1/10, A61K 8/26, A61Q 5/06, A61K 8/22, A61K 8/892, A61Q 19/08, A61K 8/34, A61K 8/58

(54) **Composition cosmétique comprenant des composés ayant une structure cage**

(30) Priorité: 10.09.2004 FR 0452017
(71) Demandeur: L'OREAL, 75008 Paris (FR)
(72) Inventeur: Mathonneau, Estelle, 75010, Paris (FR)
(74) Mandataire: Catherine, Alain

(57) **Abrégé**

L'invention concerne une composition cosmétique comprenant, dans un milieu cosmétiquement acceptable, au moins un composé ayant une structure cage ouverte ou fermée, le ou lesdits composés comprenant au moins 4 atomes d'un élément choisi parmi les éléments des colonnes 3 à 13 de la classification périodique, et au moins 4 atomes d'oxygène, lesdits atomes dudit élément étant liés uniquement à des atomes d'oxygène et à un ou plusieurs substituants identiques ou différents.

## Description

L'invention concerne une composition cosmétique comprenant, dans un milieu cosmétiquement acceptable, au moins un composé ayant une structure cage comprenant des atomes d'un élément choisi parmi les éléments des colonnes 3 à 13 de la classification périodique des éléments (MERCK INDEX, 12° édition), ainsi que l'utilisation d'une telle composition pour la mise en forme des cheveux. L'invention concerne également l'utilisation d'un composé ayant une structure cage pour améliorer l'effet tenseur de produits cosmétiques pour la peau, pour améliorer la résistance des polymères utilisés dans des vernis, et pour améliorer le recourbement des cils dans des produits de mascara.

Les produits de coiffage permettant de fixer la coiffure de façon non permanente sont généralement des gels, des solutions ou des sprays contenant un polymère fixant.

Mais ces produits ne permettent qu'une tenue de la coiffure limitée dans le temps, même si on augmente la proportion de polymère fixant. De plus, ces produits conduisent à un toucher collant sur les cheveux.

Les produits de permanente permettent une mise en forme durable de la chevelure.

Généralement, la technique utilisée pour obtenir une déformation permanente des cheveux consiste, dans un premier temps, à réaliser l'ouverture des liaisons disulfures -S-S- de la kératine (cystine) en appliquant sur les cheveux préalablement mis sous tension (bigoudis et autres) une composition réductrice (étape de réduction) puis, de préférence après avoir rincé la chevelure ainsi traitée, à reconstituer dans un second temps lesdites liaisons disulfures en appliquant sur les cheveux toujours sous tension une composition oxydante (étape d'oxydation, dite aussi de fixation) de façon à donner finalement aux cheveux la forme recherchée.

La nouvelle forme imposée aux cheveux par un traitement chimique tel que ci-dessus est durable dans le temps et résiste notamment à l'action des lavages à l'eau ou par shampooings.

Cependant, une telle technique ne donne pas entièrement satisfaction. En effet, cette technique est très efficace pour modifier la forme des cheveux, mais très dégradante pour les fibres capillaires, et altère en particulier le toucher des cheveux.

Par ailleurs, on attend de certains produits cosmétiques destinés à être appliqués sur la peau qu'ils aient un effet tenseur. Les polymères utilisés dans les compositions cosmétiques doivent avoir une certaine élasticité, mais aussi une bonne résistance mécanique.

Or, les polymères utilisés actuellement sont généralement élastiques mais souvent peu résistants mécaniquement.

On connaît du document GB 1 556 376 l'utilisation d'une composition comprenant un composé (O=Al-X)ₙ, ou X est un groupe carboxylique, pour former des films flexibles et qui adhèrent bien sur la peau. Mais ces composés à base d'aluminium ne présentent pas une structure cage.

La demanderesse a découvert de façon surprenante que l'utilisation de composés ayant une structure cage à base d'atomes d'un élément des colonnes 3 à 13 de la classification périodique permet de renforcer les propriétés viscoélastiques et mécaniques des polymères utilisés dans les compositions cosmétiques, en particulier d'améliorer la fixation de la coiffure ainsi que sa tenue dans le temps, d'améliorer l'effet tenseur des compositions cosmétiques, d'améliorer la résistance des polymères utilisés dans des applications de vernis, et d'améliorer la capacité de compositions de mascara à entraîner le recourbement des cils.

Ainsi, l'invention concerne une composition cosmétique comprenant, dans un milieu cosmétiquement acceptable, au moins un composé ayant une structure cage ouverte ou fermée, le ou lesdits composés comprenant au moins 4 atomes d'un élément choisi parmi les éléments des colonnes 3 à 13 de la classification périodique, et au moins 4 atomes d'oxygène, lesdits atomes dudit élément étant liés uniquement à des atomes d'oxygène et à un ou plusieurs substituants identiques ou différents.

Par composé ayant une structure cage, on entend au sens de la présente invention un composé dont l'agencement des atomes dudit élément et des atomes d'oxygène, et l'agencement des liaisons entre les atomes dudit élément et des atomes d'oxygène forment au moins 3 faces, de préférence 4 faces, d'au moins un polyèdre, les sommets des faces étant constitués par lesdits atomes, et les arêtes des faces étant constituées par lesdites liaisons.

Par cage fermée, on entend au sens de la présente invention une cage dont toutes les arêtes du ou desdits polyèdres constituent une liaison entre un atome dudit élément et un atome d'oxygène.

Par cage ouverte, on entend au sens de la présente invention une cage dont certaines arêtes du ou desdits polyèdres ne constituent pas une liaison entre un atome dudit élément et un atome d'oxygène.

De préférence, le ou lesdits composés comprennent entre 4 et 20, de préférence entre 4 et 12, mieux 6, 7, 8, 9, 10 ou 12 atomes dudit élément des colonnes 3 à 13.

De préférence, le ou lesdits composés ont une structure cage fermée.

Selon un mode de réalisation particulier, la structure cage du ou desdits composés forme un feuillet.

Par feuillet, on entend au sens de la présente invention un ensemble de polyèdres ayant 2 à 2 une face commune et ordonnés selon un plan.

Ainsi, par exemple, lorsque les polyèdres sont des cubes, un composé ayant une structure cage formant un feuillet peut présenter la géométrie suivante :

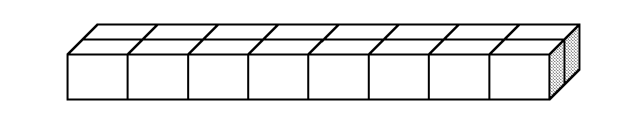

Selon un autre mode de réalisation particulier,la structure cage du ou desdits composés forme un groupement.

Par groupement, on entend au sens de la présente invention l'empilement d'au moins deux feuillets selon une direction n'appartenant pas aux plans desdits feuillets.

Comme expliqué précédemment, le ou les composés ayant une structure cage comprennent au moins 4 atomes d'un élément choisi parmi les éléments des colonnes 3 à 13 de la classification périodique.

De préférence, l'élément est choisi parmi les éléments des colonnes 8 à 13, de préférence le fer, l'aluminium et le gallium.

Lorsque l'élément est l'aluminium, le ou les composés sont nommés alumoxanes.

Selon leur valence, les atomes dudit élément des colonnes 3 à 13 du tableau périodique peuvent être liés à un ou plusieurs substituants identiques ou différents.

Les substituants peuvent être choisis parmi l'hydrogène ou parmi les groupes hydroxyle, alkyle, alkylène, alcényle, aryle, acyle, alcoxy, de préférence méthoxy, hydride, ester, carboxyle, acrylate, alkyl acrylate, alcool, aldéhyde, amine, alkylamine, silanol, les groupes contenant une ou plusieurs fonctions amines, les groupes siloxane, les groupes contenant un ou plusieurs groupes siloxane, les groupes silicone ou les groupes contenant un ou plusieurs groupes silicones, les groupe silane ou les groupes contenant un ou plusieurs groupes silanes, les groupes contenant un ou plusieurs atomes de fluor, de soufre ou de phosphore, les groupes SO₂, CO₂X, SO₃X ou X est un atome d'hydrogène, un méthyl ou un éthyl, les groupes alpha-oléphines, époxyde, azo, diazo, halogène, les groupes cycliques pouvant donner lieu à une isomérisation par ouverture de cycle, la silice moléculaire, les groupe nitrile, thiol, et les polymères, de préférence les polyacrylates, les résines époxy, les résines phénol-formaldéhyde, les polyamides, les polyesters, les polyimides, les polycarbonates, les polyuréthannes et les polymères quinone amine.

On peut citer en particulier les groupes phénol, amine quaternaire, haloalkyle, méthacrylate, halosilane, styrène et norbornényle et ter-butyle.

Les substituants préférés sont les substituants permettant une meilleure solubilisation des composés ayant une structure cage dans le milieu physiologiquement acceptable, les substituants ayant une affinité avec un des composants du cheveu ou de la peau, et les substituants cationiques.

Lorsque le ou les susbtituants sont sous forme de polymère, le polymère peut être obtenu :
- soit par polymérisation de composés ayant une structure cage, lesdits composés étant fonctionnalisés par au moins deux substituants réactifs chimiquement pour permettre la polymérisation,
- soit par greffage sur le ou lesdits composés d'un ou plusieurs substituants polymériques tels que décrits précédemment.

Ainsi, par exemple, des polymères à base d'aluminoxane sont décrits dans le document WO 00/09578.

Lorsque le ou les composés sont des alumoxanes, ils peuvent être représentés par les formules suivantes : [(R)Al(O)]ₙ ou bien R'[(R")Al(O)]ₙAlR"'₂, ou R, R', R" et R"' sont des substituants tels que décrits précédemment, et ou n est un entier compris entre 4 et 20.

De même, lorsque le ou les composés sont à base de gallium, ils peuvent être représentés par les formules suivantes : [(R)Ga(O)]ₙ ou bien R[(R')Ga(O)]ₙAIR"'₂, ou R, R', R" et R"' sont des substituants tels que décrits précédemment, et ou n est un entier compris entre 4 et 20.

On peut citer en particulier les composés suivants :
- le [(^{t}Bu)M(µ₃-O)]₆ de structure 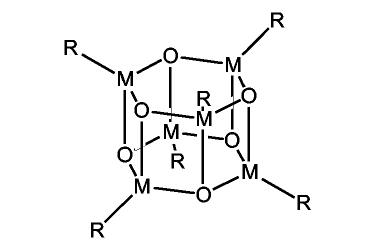 où R représente le groupe terbutyle,
- le [(^{t}Bu)M(µ₃-O)]₇ de structure 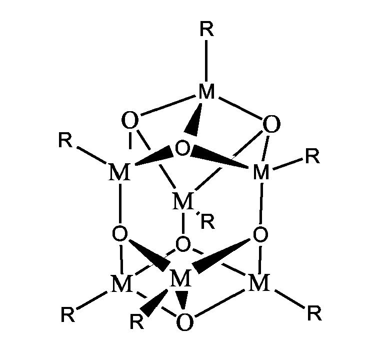 où R représente le groupe terbutyle,
- le [(^{t}Bu)M(µ₃-O)]₈ de structure 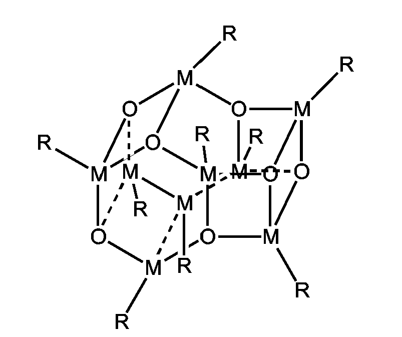 où R représente le groupe terbutyle,
- le [(^{t}Bu)M(µ₃-O)]₉ de structure 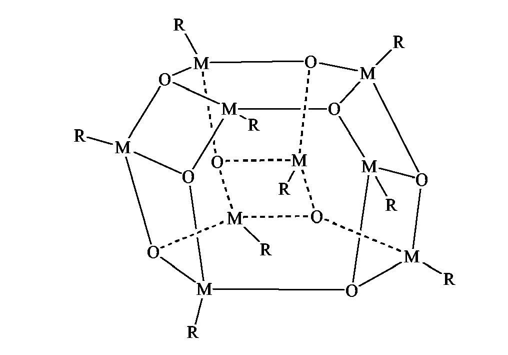 où R représente le groupe terbutyle,
- le [(^{t}Bu)M(µ₃-O)]₁₀ de structure 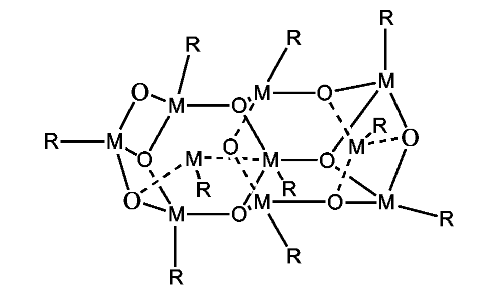 où R représente un groupe terbutyle,
- le [(^{t}Bu)M(µ₃-O)]₁₂ de structure dodécaédrique 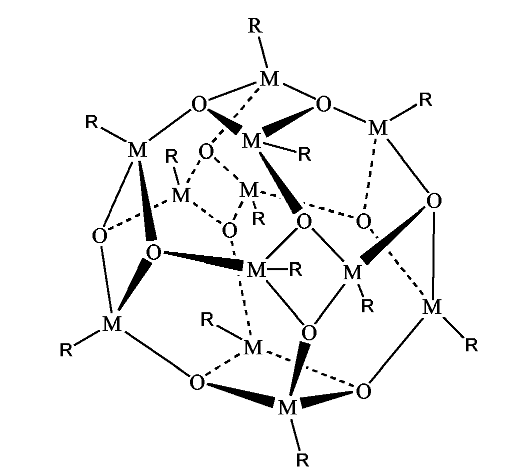 où R représente le groupe terbutyle,
   M représentant AI ou Ga.

Ces composés sont des composés à structure cage fermée.

Comme exemple de composés alumoxanes à structure cage ouverte, on peut citer :
- le [^{t}Bu₇Al₅(µ₃-O)₃(µ-OH)₂], à structure cage ouverte, de formule : 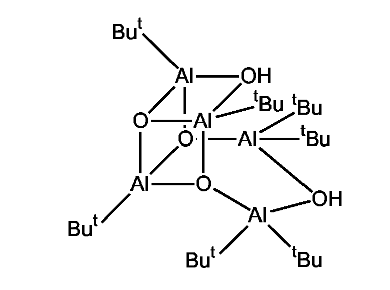
- le [^{t}Bu₈Al₆(µ₃-O)₄(µ-OH)₂], à structure cage ouverte, de formule :

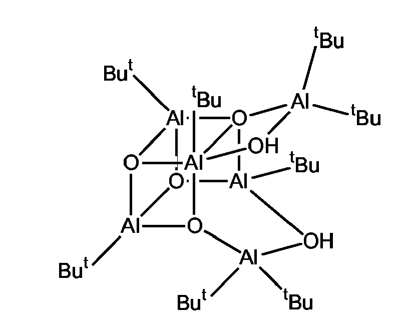

La synthèse des cages à base d'aluminium est décrite par exemple dans les références S.Pasynkiewicz, *Polyhedron,* **9(2/3),** (1990), 429-453, C.T.Vogelson, A.R Barron, *Journal of Non-cristalline Solids,* **290(2,3),** (2001) 216-223, Callender, R.L., C.J.Harlan, N.M.Shapiro, C.D.Jones, D.B.Macqueen, D.L.Callahan, M.R.Wiesner, R.Cook and A.R Barron, *Chemistry of Materials,* **9(11) ,** (1997), 2418-2433.

La synthèse des cages à base de fer est décrite par exemple dans les références J.Rose, M.Cortalezzi-Fidalgo, S.Moustier, C.Magnetto, C.Jones, A.Barron, M.Wiesner, JY Bottero, *Chemistry of Materials,* **14(2),** (2002), 621-628.

La synthèse des cages de gallium est décrite dans la référence: H.W.Roesky, *Acc. Chem.Res.,* **34** (2001) 201-211

La composition cosmétique comprend généralement de 0,00001 à 20%, de préférence de 0,0001 à 10%, mieux de 0,001 à 5%, en poids de composé(s) ayant une structure cage.

Le ou les constituants de la composition selon l'invention doivent être placés dans un milieu cosmétiquement acceptable.

Pour cela, on choisit généralement un ou plusieurs solvants classiquement utilisés dans les compositions cosmétiques. On peut citer en particulier l'eau, les alcools inférieurs en C₁-C₄, de préférence l'éthanol et l'isopropanol, les cétones en C₃-C₆, de préférence l'acétone et la méthyléthylcétone, les esters en C₃-C₆, de préférence l'acétate d'éthyle et l'acétate de butyle, les éthers en C₂-C₆, de préférence le diéthoxyéthane et le diméthoxyéthane, et les alcanes en C₆-C₁₀.

Outre le ou les composés ayant une structure cage, la composition cosmétique selon l'invention peut comprendre un ou plusieurs polymères fixant cationique, anionique, amphotère ou non ionique.

Par polymère fixant on entend tout polymère susceptible de conférer une forme à une chevelure ou de permettre le maintien d'une forme de la chevelure.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5 000 000 et de préférence entre 1000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs suivants : 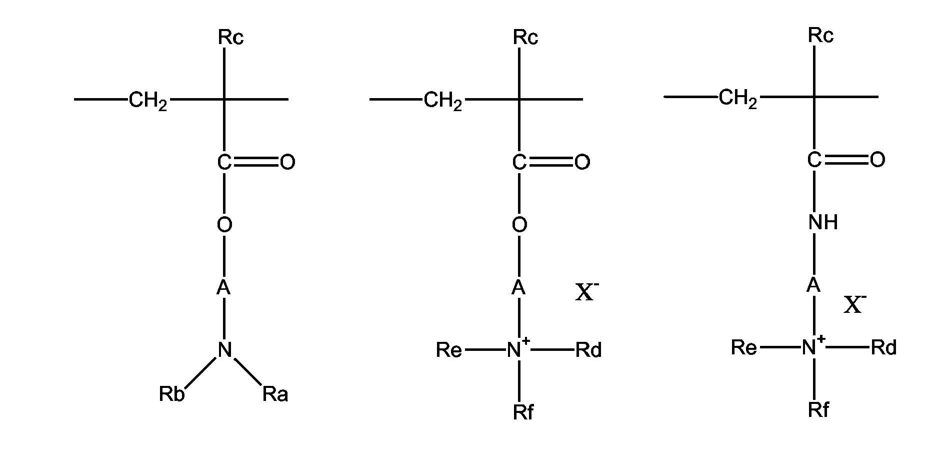 dans lesquelles
   Ra et Rb représentent chacun un atome d'hydrogène ou un groupe alkyle en C1-6, Rc désigne un atome d'hydrogène ou un radical CH3,
   Rd, Re et Rf, identiques ou différents, représentent chacun un groupe alkyle en C1-18 ou un radical benzyle,
   A est un groupe alkyle linéaire ou ramifié en C1-6 ou un groupe hydroxyalkyle en C1-4, et
   X⁻ désigne un anion méthosulfate ou halogénure tel qu'un ion chlorure
   ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieurs, des acides acryliques ou méthacryliques ou leurs esters, des vinyllactames tels que la vinylpyrrolidone ou le vinylcaprolactame, et des esters vinyliques.
   Ainsi, on peut citer parmi les copolymères de la famille (1)
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC TM par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT TM P 100 par la société CIBA GEIGY,
   - le copolymère d'acrylamide et de méthosulfate de méthacryloyloxyéthyltriméthylammonium vendu sous la dénomination RETEN TM par la société HERCULES,
   - les copolymères vinylpyrrolidone/acrylate ou méthacrylate de dialkylaminoalkyle quaternisés ou non, tels que les produits vendus sous la dénomination GAFQUAT TM par la société ISP, comme par exemple GAFQUAT TM 734 ou GAFQUAT TM 755, ou bien les produits dénommés COPOLYMER TM 845, 958 et 937. Ces polymères sont décrits en détail dans les brevets français 2.077.143 et 2.393.573,
   - les terpolymères méthacrylate de diméthylaminoéthyle/vinylca-prolactame/vinylpyrrolidone tels que le produit commercialisé sous la dénomination GAFFIX VC 713 par la société ISP, et
   - le copolymère vinylpyrrolidone/méthacrylamide de diméthyl-aminopropyle quaternisé tel que le produit vendu sous la dénomination GAFQUAT TM HS 100 par la société ISP.
(2) Les polysaccharides quaternisés décrits plus particulièrement dans les brevets américains 3.589.578 et 4.031.307 tels que les gommes de guar contenant des groupements cationiques trialkylammonium.
   De tels produits sont commercialisés notamment sous les dénominations commerciales de JAGUAR TM C13 S, JAGUAR TM C 15 et JAGUAR TM C 17 par la société MEYHALL.
(3) les copolymères quaternaires de vinylpyrrolidone et de vinylimidazole tels que les produits commercialisés par BASF sous la dénomination LUVIQUAT TM TFC,
(4) les chitosanes ou leurs sels, en particulier les acétate, lactate, glutamate, gluconate ou pyrrolidonecarboxylate de chitosane.
   On peut citer le chitosane ayant un taux de désacétylation de 90,5 % en poids vendu sous la dénomination KYTAN BRUT STANDARD TM par la société ABER TECHNOLOGIES, le pyrrolidonecarboxylate de chitosane vendu sous la dénomination KYTAMER TM PC par la société AMERCHOL.
(5) les dérivés de cellulose cationiques tels que les copolymères de cellulose ou les dérivés de cellulose greffés avec un monomère hydrosoluble comportant un groupe ammonium quaternaire et décrits notamment dans le brevet US 4 131 576 tels que les hydroxyalkylcelluloses, comme les hydroxyméthylhydroxyéthyl- ou hydroxypropylcelluloses greffées notamment avec un sel de méthacryloyloxyéthyltriméthylammonium, de méthacrylamidopropyltriméthylammonium ou de diméthyldiallylammonium.

Les produits commercialisés répondant à cette définition sont plus particulièrement les produits vendus sous la dénomination CELQUAT TM L 200 et CELQUAT TM H 100 par la société NATIONAL STARCH.

Les polymères fixants anioniques généralement utilisés sont des polymères comportant des groupements dérivés d'un acide carboxylique, sulfonique ou phosphorique et ont un poids moléculaire moyen en poids compris entre environ 500 et 5 000 000.

Les groupements acide carboxylique sont apportés par des monomères insaturés contenant une ou deux fonctions acide carboxylique, tels que ceux répondant à la formule: 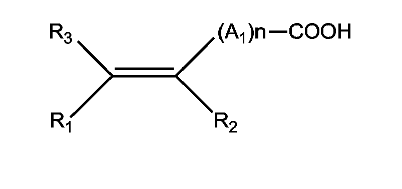
dans laquelle n est un nombre entier de 0 à 10, A1 désigne un groupement méthylène, éventuellement relié à l'atome de carbone du groupement insaturé ou du groupement méthylène voisin, lorsque n est supérieur à 1, par l'intermédiaire d'un hétéroatome tel que oxygène ou soufre, R3 désigne un atome d'hydrogène, un groupement phényle ou benzyle, R1 désigne un atome d'hydrogène, un groupement alkyle inférieur ou carboxyle, et R2 désigne un atome d'hydrogène, un groupement alkyle inférieur, un groupement -CH2-COOH, phényle ou benzyle.

Dans la formule précitée un radical alkyle inférieur désigne de préférence un groupement ayant de 1 à 4 atomes de carbone et en particulier un groupe méthyle ou éthyle.

Les polymères fixants anioniques carboxylés préférés selon l'invention sont:
A) les homo- ou copolymères d'acide acrylique ou méthacrylique ou leurs sels et en particulier les produits commercialisés sous les dénominations VERSICOL TM E ou K par la société ALLIED COLLOID, et sous la dénomination ULTRAHOLD TM par la société BASF ; les copolymères d'acide acrylique et d'acrylamide commercialisés sous forme de sel de sodium sous les dénominations RETEN TM 421, 423 ou 425 par la Société HERCULES ; les sels de sodium des acides polyhydroxycarboxyliques.
B) les copolymères d'acide acrylique ou d'acide méthacrylique et d'un monomère monoéthylénique tel que l'éthylène, le styrène, les esters vinyliques, les esters d'acide acrylique ou méthacrylique.
   Ces copolymères peuvent être greffés sur un polyalkylène glycol tel que le polyéthylèneglycol et sont éventuellement réticulés.
   De tels polymères sont décrits en particulier dans le brevet français FR 1 222 944 et dans la demande de brevet allemand DE 2 330 956. On peut notamment citer les copolymères comportant dans leur chaîne un motif acrylamide éventuellement N-alkylé et/ou hydroxyalkylé, tels que ceux décrits dans les demandes de brevet luxembourgeois LU 75370 et LU 75371 ou proposés sous la dénomination QUADRAMER TM par la Société AMERICAN CYANAMID.
   On peut également citer les copolymères d'acide acrylique et de méthacrylate d'alkyle en C1-C4, et les terpolymères de vinylpyrrolidone, d'acide (méth)acrylique et de (méth)acrylate d'alkyle en C1-C20, par exemple de lauryle (ACRYLDONE TM LM de la société ISP), de tertiobutyle (LUVIFLEX TM VBM 70 commercialisé par BASF) ou de méthyle (STEPANHOLD TM EXTRA commercialisé par STEPAN), et les terpolymères acide méthacrylique/acrylate d'éthyle/acrylate de tertiobutyle tel que le produit commercialisé sous la dénomination LUVIMER TM 100 P par la société BASF.
C) les copolymères dérivés d'acide crotonique tels que ceux comportant dans leur chaîne des motifs acétate ou propionate de vinyle et éventuellement d'autres monomères tels que les esters allylique, méthallylique ou vinylique d'un acide carboxylique saturé, linéaire ou ramifié, à longue chaîne hydrocarbonée comportant au moins 5 atomes de carbone, ces polymères pouvant éventuellement être greffés et réticulés, ou encore les esters vinylique, allylique ou méthallylique d'un acide carboxylique alpha - ou beta -cyclique.
   De tels polymères sont décrits, entre autres, dans les brevets français FR 1 222 944, FR 1 580 545, FR 2 265 782, FR 2 265 781, FR 1 564 110 et FR 2 439 798.
   On peut citer à titre d'exemples de produits commerciaux entrant dans cette classe les résines 28-29-30, 26-13-14 et 28-13-10 commercialisées par la société NATIONAL STARCH.
D) les copolymères dérivés d'acides ou d'anhydrides carboxyliques monoinsaturés en C4-C8 choisis parmi:
   - les copolymères comprenant
      (i) un ou plusieurs acides ou anhydrides maléique, fumarique, itaconique et
      (ii) au moins un monomère choisi parmi les esters vinyliques, les éthers vinyliques, les halogénures vinyliques, les dérivés phénylvinyliques, l'acide acrylique et ses esters, les fonctions anhydride de ces copolymères étant éventuellement monoestérifiées ou monoamidifiées;

   De tels polymères sont décrits en particulier dans les brevets US 2,047,398, US 2,723,248, US 2,102,113 et GB 839,805 et notamment ceux commercialisés sous les dénominations GANTREZ AN ou ES, AVANTAGE TM CP par la société ISP;
   - les copolymères comprenant (i) un ou plusieurs anhydrides maléique, citraconique ou itaconique et (ii) un ou plusieurs monomères choisis parmi les esters allyliques ou méthallyliques comportant éventuellement un ou plusieurs groupements acrylamide, méthacrylamide, alpha -oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

   Ces polymères sont par exemple décrits dans les brevets français FR 2 350 384 et FR 2 357 241 de la demanderesse.
E) les polyacrylamides comportant des groupements carboxylate.
   Les groupements anioniques des polymères fixants anioniques de la présente invention peuvent également être des groupes acide sulfonique apportés par des motifs vinylsulfonique, styrènesulfonique, naphtalènesulfonique ou acrylamidoalkylsulfonique.
   Ces polymères à groupes acide sulfonique sont notamment choisis parmi:
   - les sels de poly(acide vinylsulfonique) ayant un poids moléculaire moyen en poids compris entre environ 1000 et 100 000 ainsi que les copolymères d'acide vinylsulfonique et d'un comonomère insaturé tel que l'acide acrylique, l'acide méthacrylique, les esters de ces acides, l'acrylamide, les dérivés d'acrylamide, les éthers vinyliques et la vinylpyrrolidone ;
   - les sels de poly(acide styrènesulfonique). On peut citer à titre d'exemple deux sels de sodium ayant un poids moléculaire moyen en poids d'environ 500 000 et d'environ 100 000 commercialisés respectivement sous les dénominations FLEXAN TM 500 et FLEXAN TM 130 par la société NATIONAL STARCH. Ces composés sont décrits dans le brevet FR 2 198 719 ;
   - les sels de poly(acide acrylamidosulfonique), tels que ceux mentionnés dans le brevet US 4,128,631 et plus particulièrement le poly(acide acrylamidoéthylpropanesulfonique) commercialisé sous la dénomination COSMEDIA POLYMER TM HSP 1180 par la société HENKEL.

Selon l'invention, les polymères fixants anioniques sont de préférence choisis parmi les copolymères d'acide acrylique tels que les terpolymères acide acrylique/acrylate d'éthyle/N-tertiobutylacrylamide commercialisés notamment sous la dénomination ULTRAHOLD STRONG TM par la société BASF, les copolymères dérivés d'acide crotonique tels que les terpolymères acétate de vinyle/tertio-butyl benzoate de vinyle/acide crotonique et les terpolymères acide crotonique/acétate de vinyle/néododécanoate de vinyle commercialisés notamment sous la dénomination Résine 28-29-30 par la société NATIONAL STARCH, les copolymères dérivés d'acide ou d'anhydride maléique, fumarique ou itaconique et contenant comme comonomères des esters vinyliques, des éthers vinyliques, des halogénures vinyliques, des dérivés phénylvinyliques, l'acide acrylique et les esters d'acide acrylique, tels que les copolymères méthylvinyléther/anhydride maléique monoestérifié, commercialisés par exemple sous la dénomination GANTREZ TM par la société ISP, les copolymères d'acide méthacrylique et de méthacrylate de méthyle commercialisés sous la dénomination EUDRAGIT TM L par la société ROHM PHARMA, les copolymères acide méthacrylique/méthacrylate de méthyle/acrylate d'alkyle en C1-4 /acide acrylique ou méthacrylate d'hydroxyalkyle en C1-4, commercialisés sous la dénomination AMERHOLD TM DR 25 par la société AMERCHOL, ou sous la dénomination ACUDYNE TM 255 par la société ROHM & HAAS, les copolymères d'acide méthacrylique et d'acrylate d'éthyle commercialisés sous la dénomination LUVIMER TM MAEX ou MAE par la société BASF et les copolymères acétate de vinyle/acide crotonique et les copolymères acétate de vinyle/acide crotonique greffés par du polyéthylèneglycol commercialisés sous la dénomination ARISTOFLEX TM A par la société BASF.

Les polymères fixants amphotères utilisables pour la présente l'invention sont choisis notamment parmi les polymères comportant des motifs B et C répartis statistiquement dans la chaîne polymère, où B désigne un motif dérivant d'un monomère comportant au moins un atome d'azote basique et C désigne un motif dérivant d'un monomère comportant un ou plusieurs groupements acide carboxylique ou acide sulfonique. Les polymères fixants amphotères peuvent également comporter des motifs zwittérioniques de type carboxybétaïne ou sulfobétaïne. Il peut également s'agir de polymères à chaîne principale cationique comportant des groupements amine primaire, secondaire, tertiaire ou quaternaire, parmi lesquels au moins un porte, par l'intermédiaire d'un radical hydrocarboné, un groupement acide carboxylique ou acide sulfonique. Les polymères fixant amphotères peuvent encore avoir une chaîne anionique dérivée d'acides dicarboxyliques alpha , beta -insaturés dont l'un des groupements carboxyle a été amené à réagir avec une polyamine comportant un ou plusieurs groupements amine primaire ou secondaire.

Les polymères fixants amphotères répondant à la définition donnée ci-dessus sont choisis en particulier parmi les polymères suivants:
(1) Les polymères résultant de la copolymérisation d'un monomère vinylique portant un groupement acide carboxylique tel que l'acide acrylique, l'acide méthacrylique, l'acide maléique, l'acide alpha -chloroacrylique, et d'un monomère vinylique contenant au moins une fonction basique tel que les méthacrylate et acrylate de dialkylaminoalkyle ou les dialkylaminoalkyl(méth)acrylamides. De tels composés sont décrits par exemple dans le brevet américain US 3,836,537.
(2) Les polymères comportant des motifs dérivés :
   (a) d'au moins un monomère choisi parmi les acrylamides ou méthacrylamides N-alkylés,
   (b) d'au moins un comonomère contenant une ou plusieurs fonctions acide carboxylique, et
   (c) d'au moins un comonomère basique tel que des esters à substituants amine primaire, secondaire, tertiaire ou quaternaire d'acide acrylique et d'acide méthacrylique, et le produit de quaternisation du méthacrylate de diméthylaminoéthyle avec le sulfate de diméthyle ou diéthyle.

   Les acrylamides ou méthacrylamides N-alkylés (a) préférés sont ceux portant des radicaux alkyle en C2-12, tels que le N-éthylacrylamide, le N-tertiobutylacrylamide, le N-tertio-octylacrylamide, le N-octylacrylamide, le N-décylacrylamide, le N-dodécylacrylamide ainsi que les méthacrylamides correspondants.
   Les comonomères à groupe acide carboxylique (b) sont choisis plus particulièrement parmi les acides acrylique, méthacrylique, crotonique, itaconique, maléique, fumarique ainsi que parmi les monoesters d'alkyle en C1-4 des acides ou des anhydrides maléique ou fumarique.
   Les comonomères basiques (c) préférés sont le méthacrylate d'aminoéthyle, le méthacrylate de butylaminoéthyle, le méthacrylate de N,N'-diméthylaminoéthyle et le méthacrylate de N-tertiobutylaminoéthyle.
   On utilise en particulier les copolymères dont la dénomination CTFA (4<ème> Ed., 1991) est "Octylacrylamide/acrylates/butylaminoethyl-methacrylate copolymer", tels que les produits commercialisés sous la dénomination AMPHOMER TM ou LOVOCRYL TM 47 par la société NATIONAL STARCH.
(3) Les polyaminoamides réticulés et alcoylés, dérivés en partie ou en totalité de polyaminoamides de formule générale:

   (II) -[C(=O)-R4-C(=O)-Z-]-

   dans laquelle R4 représente un radical divalent dérivé d'un acide dicarboxylique saturé, d'un acide aliphatique mono- ou dicarboxylique à double liaison éthylénique, d'un ester d'alkyle en C1-6 de ces acides ou d'un radical dérivant de l'addition de l'un quelconque desdits acides sur une amine bis primaire ou bis secondaire, et Z désigne un radical d'une polyalkylène-polyamine bis-primaire, mono- ou bis-secondaire, et de préférence représente:
   a) dans les proportions de 60 à 100 % en moles, le radical

      (III) -NH-[(CH₂)ₓ-NH]ₚ-

      où x=2 et p=2 ou 3, ou bien x =3 et p=2
      ce radical dérivant de la diéthylènetriamine, de la triéthylènetétraamine ou de la dipropylènetriamine;
   b) dans les proportions de 0 à 40 % en moles le radical de formule (III) dans lequel x = 2 et p = 1, dérivé de l'éthylènediamine, ou le radical 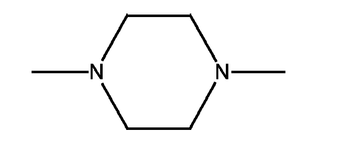 dérivé de la pipérazine:
   c) dans les proportions de 0 à 20 % en moles le radical -NH-(CH2)6-NH- dérivé de l'hexaméthylènediamine, ces polyaminoamines étant réticulées par addition de 0,025 à 0,35 mole par mole de groupement amine, d'un agent réticulant bifonctionnel choisi parmi les épihalohydrines, les diépoxydes, les dianhydrides, les composés di-insaturés, et alcoylés par l'acide acrylique, l'acide chloracétique ou une alcane-sultone.

   Les acides carboxyliques saturés sont choisis de préférence parmi les acides ayant 6 à 10 atomes de carbone tels que l'acide adipique, l'acide triméthyl-2,2,4-adipique et triméthyl-2,4,4-adipique, l'acide téréphtalique, les acides à double liaison éthylénique comme par exemple les acides acrylique, méthacrylique, itaconique.
   Les alcane-sultones utilisées dans l'alcoylation sont de préférence la propanesultone ou la butanesultone.
   Les sels des agents d'alcoylation sont de préférence les sels de sodium ou de potassium.
(4) Les polymères comportant des motifs zwittérioniques de formule: 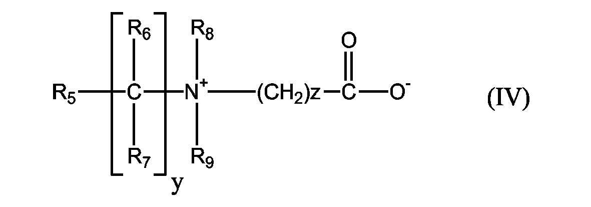
   dans laquelle R5 désigne un groupement insaturé polymérisable tel qu'un groupement acrylate, méthacrylate, acrylamide ou méthacrylamide, y et z représentent chacun un nombre entier de 1 à 3, R6 et R7 représentent chacun indépendamment un atome d'hydrogène ou un groupe méthyle, éthyle ou propyle, R8 et R9 représentent chacun indépendamment un atome d'hydrogène ou un radical alkyle, le nombre total d'atomes de carbone dans R8 et R9 ne dépassant pas 10.
   Les polymères comprenant de tels motifs de formule (IV) peuvent comporter en outre des motifs dérivés de monomères non zwittérioniques tels que l'acrylate ou le méthacrylate de diméthyle ou de diéthylaminoéthyle, les acrylates ou méthacrylates d'alkyle, les acrylamides ou méthacrylamides ou l'acétate de vinyle.
   A titre d'exemple, on peut citer le copolymère méthacrylate de méthyle/méthacrylate de diméthylcarboxyméthylammonioéthyle tel que le produit commercialisé sous la dénomination DIAFORMER TM Z301 par la société SANDOZ.
(5) Les polymères dérivés du chitosane comportant des motifs monomères répondant aux formules suivantes: 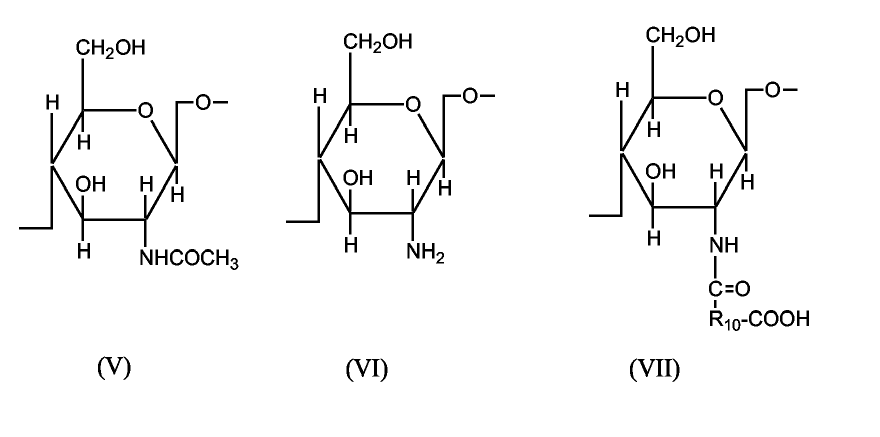 le motif de formule (V) étant présent dans des proportions comprises entre 0 et 30 %, le motif de formule (VI) dans des proportions comprises entre 5 et 50 % et le motif de formule (VII) dans des proportions comprises entre 30 et 90 %, étant entendu que dans ce motif (VII), R10 représente un radical de formule: 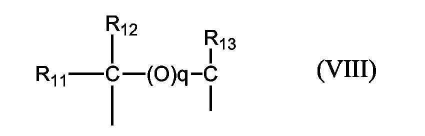 dans laquelle
   si q = 0, alors R11, R12 et R13, identiques ou différents, représentent chacun un atome d'hydrogène, un groupe méthyle, hydroxyle, acétoxy ou amino, un groupe monoalcoylamine ou dialcoylamine éventuellement interrompu par un ou plusieurs atomes d'azote et/ou éventuellement substitués par un ou plusieurs groupes amine, hydroxyle, carboxyle, alcoylthio ou sulfo, un groupe alcoylthio dont le groupe alcoyle porte un reste amino, l'un au moins des radicaux R11, R12 et R13 étant dans ce cas un atome d'hydrogène; ou si q = 1, alors R11, R12 et R13 représentent chacun un atome d'hydrogène, ainsi que les sels formés par ces composés avec des bases ou des acides.
(6) Les polymères obtenus par N-carboxyalkylation du chitosane, comme le N-carboxyméthylchitosane ou le N-carboxybutylchitosane commercialisé sous la dénomination EVALSAN TM par la société JAN DEKKER.
(7) Les polymères répondant à la formule générale (IX) 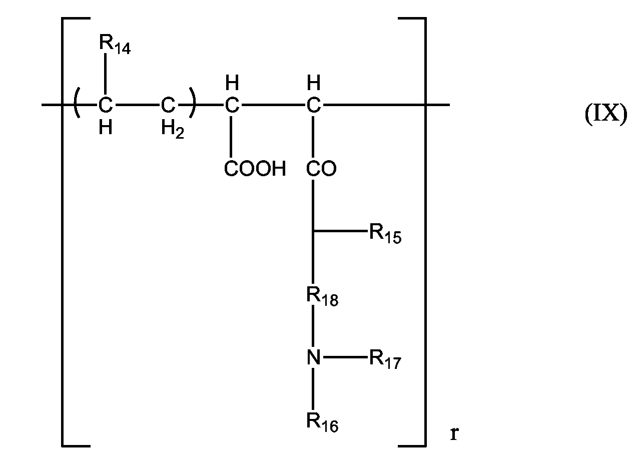 décrits notamment dans le brevet FR 1 400 366, dans laquelle R14 représente un atome d'hydrogène ou un radical CH30, CH3CH20 ou phényle, R15 désigne un atome d'hydrogène ou un radical alkyle inférieur tel que méthyle ou éthyle, R16 désigne un atome d'hydrogène ou un radical alkyle inférieur tel que méthyle ou éthyle, R17 désigne un radical alkyle inférieur tel que méthyle ou éthyle ou un radical répondant à la formule: - R18-N(R16)2, R18 représentant un groupement -CH2-CH2-, -CH2-CH2-CH2-, -CH2-CH(CH3)-, et R16 ayant les significations mentionnées ci-dessus, ainsi que les homologues supérieurs de ces radicaux contenant jusqu'à 6 atomes de carbone.
(8) Les polymères amphotères du type -D-X-D-X- choisis parmi:
   (a) les polymères obtenus par action d'acide chloroacétique ou de chloroacétate de sodium sur les composés comportant au moins un motif de formule: (X) -D-X-D-X-D-
      où D désigne un radical 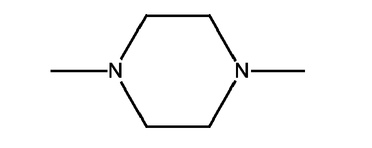 et X désigne le symbole E ou E', E ou E' identiques ou différents désignant un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote ou de soufre, 1 à 3 cycles aromatiques et/ou hétérocycliques, les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne,
   b) les polymères de formule: (X') -D-X'-D-X'-
      où D désigne un radical 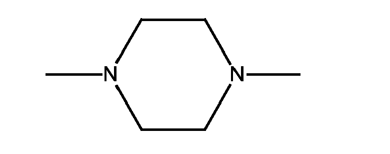 et X' désigne le symbole E ou E' et au moins une fois E', E ayant la signification indiquée ci-dessus et E' étant un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloroacétique ou du chloroacétate de soude.
   (9) Les copolymères alkyl(C1-5)vinyléther/anhydride maléique modifiés partiellement par semi-amidification avec une N,N-dialkylaminoalkylamine telle que la N,N-diméthylaminopropylamine, ou par semiestérification avec une N,N-dialcanolamine. Ces copolymères peuvent également comporter d'autres comonomères vinyliques tels que le vinylcaprolactame.

Les polymères fixants amphotères préférés selon l'invention sont ceux de la famille (3) décrite ci-dessus, tels que ceux dont la dénomination CTFA est "Octylacrylamide/acrylates/butylaminoethyl-methacrylate copolymer". On peut citer à titre d'exemple les produits commercialisés sous les dénominations AMPHOMER TM , AMPHOMER TM LV 71 ou LOVOCRYL TM 47 par la société NATIONAL STARCH.

D'autres polymères fixants amphotères préférés sont ceux de la famille (4), comme par exemple les copolymères de méthacrylate de méthyle et de méthacrylate de diméthylcarboxyméthylammonioéthyle, commercialisés par exemple sous la dénomination DIAFORMER TM Z301 par la société SANDOZ.

Les polymères fixants anioniques ou amphotères peuvent, si nécessaire, être partiellement ou totalement neutralisés. Les agents de neutralisation sont par exemple la soude, la potasse, l'amino-2-méthylpropanol, la monoéthanolamine, la triéthanolamine ou la triisopropanolamine, les acides minéraux ou organiques tels que l'acide chlorhydrique ou l'acide citrique.

Les polymères fixants non ioniques utilisables selon la présente invention sont choisis par exemple parmi :
- les homopolymères de vinylpyrrolidone,
- les copolymères de vinylpyrrolidone et d'acétate de vinyle,
- les polyalkyloxazolines telles que les polyéthyloxazolines proposées par la société DOW CHEMICAL sous les dénominations PEOX TM 50 000, PEOX TM 200 000 et PEOX TM 500 000,
- les homopolymères d'acétate de vinyle tels que le produit proposé sous le nom de APPRETAN TM EM par la société HOECHST ou le produit proposé sous le nom de RHODOPAS TM A 012 par la société RHONE POULENC,
- les copolymères d'acétate de vinyle et d'esters acryliques tels que le produit proposé sous la dénomination RHODOPAS TM AD 310 par RHONE POULENC,
- les copolymères d'acétate de vinyle et d'éthylène tels que le produit proposé sous le nom de APPRETAN TM TV par la société HOECHST,
- les copolymères d'acétate de vinyle et d'ester maléique par exemple de maléate de dibutyle tels que le produit proposé sous le nom de APPRETAN TM MB EXTRA par la société HOECHST,
- les copolymères d'éthylène et d'anhydride maléique,
- les homopolymères d'acrylates d'alkyle et les homopolymères de méthacrylates d'alkyle tels que le produit proposé sous la dénomination MICROPEARL TM RQ 750 par la société MATSUMOTO ou le produit proposé sous la dénomination LUHYDRAN TM A 848 S par la société BASF,
- les copolymères d'esters acryliques tels que par exemple les copolymères d'acrylates d'alkyle et de méthacrylates d'alkyle tels que les produits proposés par la société ROHM & HAAS sous les dénominations PRIMAL AC-261 K et EUDRAGIT NE 30 D, par la société BASF sous les dénominations ACRONAL TM 601, LUHYDRAN TM LR 8833 ou 8845, et par la société HOECHST sous les dénominations APPRETAN TM N 9213 ou N9212,
- les copolymères d'acrylonitrile et d'un monomère non ionique choisi par exemple parmi le butadiène et les (méth)acrylates d'alkyle ; on peut citer les produits proposés sous les dénominations NIPOL TM LX 531 B par la société NIPPON ZEON ou ceux proposés sous la dénomination CJ 0610 B par la société ROHM & HAAS,
- les polyuréthannes tels que les produits proposés sous les dénominations ACRYSOL TM RM 1020 ou ACRYSOL TM RM 2020 par la société ROHM & HAAS, les produits URAFLEX TM XP 401 UZ, URAFLEX TM XP et 402 UZ par la société DSM RESINS,
- les copolymères d'acrylate d'alkyle et d'uréthanne tels que le produit 8538-33 commercialisé par le société NATIONAL STARCH,
- les polyamides tels que le produit ESTAPOR TM LO 11 proposé par la société RHONE POULENC,
- les gommes de guar non ioniques chimiquement modifiées ou non modifiées.

Les gommes de guar non ioniques non modifiées sont par exemple les produits vendus sous la dénomination VIDOGUM TM GH 175 par la société UNIPECTINE et sous la dénomination JAGUAR TM C par la société MEYHALL. Les gommes de guar non ioniques modifiées utilisables selon l'invention sont de préférence modifiées par des groupements hydroxyalkyle en C1-6. On peut mentionner à titre d'exemple les groupements hydroxyméthyle, hydroxyéthyle, hydroxypropyle et hydroxybutyle.

Ces gommes de guar sont bien connues dans la technique et peuvent par exemple être préparées par réaction des oxydes d'alcènes correspondants, tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les dénominations commerciales JAGUAR TM HP8, JAGUAR TM HP60 et JAGUAR TM HP120, JAGUAR TM DC 293 et JAGUAR TM HP 105 par la société MEYHALL, ou sous la dénomination GALACTASOL TM 4H4FD2 par la société AQUALON.

Selon l'invention, on peut également utiliser, en tant que polymères fixants, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4 693 935, US 4 728 571 et US 4 972 037.

Ces polymères sont de préférence anioniques ou non ioniques.

De tels polymères sont par exemple les copolymères susceptibles d'être obtenus par polymérisation radicalaire à partir du mélange de monomères formé
a) de 50 à 90 % en poids d'acrylate de tertiobutyle,
b) de 0 à 40 % en poids d'acide acrylique,
c) de 5 à 40 % en poids d'un macromère siliconé de formule 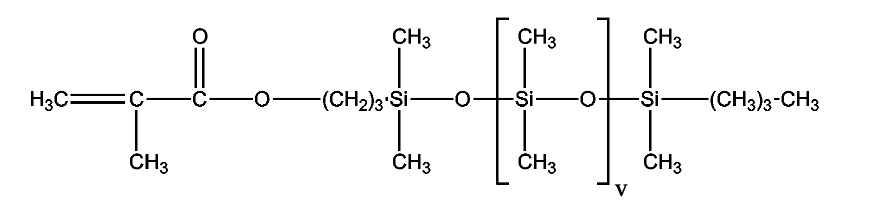
avec v étant un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser comme polymères fixants filmogènes des polyuréthannes fonctionnalisés ou non, siliconés ou non.

Les polyuréthannes particulièrement visés par la présente invention sont ceux décrits dans les brevets EP 0 751 162, EP 0 637 600, FR 2 743 297 et EP 0 648 485 de la demanderesse, ainsi que le brevet EP 0 656 021 ou WO 94/03510 de la société BASF et EP 0 619 111 de la société NATIONAL STARCH.

La composition cosmétique selon l'invention peut encore comprendre un ou plusieurs adjuvants cosmétiquement acceptables choisis parmi silicones sous forme soluble, dispersée, micro ou nano-dispersée, les agents épaississants, les agents tensio-actifs non-ioniques, anioniques, cationiques et amphotères, les agents conditionneurs, les agents adoucissants, les agents anti-mousse, les agents hydratants, les agents émollients, les plastifiants, les filtres solaires hydrosolubles et liposolubles, siliconés ou non siliconés, les colorants permanents ou temporaires, les pigments minéraux ou organiques, colorés ou non colorés, les charges minérales, les argiles, les nacres ou agents nacrants, les opacifiants, les colloïdaux, les parfums, les peptisants, les conservateurs, les céramides, et pseudo-céramides, les vitamines et les provitamines, par exemple le panthénol, les protéines, les agents séquestrants, les agents solubilisants, les agents acidifiants, les agents alcanisants, les agents anti-corrosion, les corps gras tels que les huiles végétales, animales, minérales et synthétiques, les agents réducteurs ou antioxydants, les agents oxydants, les polymères autres que les polymères fixants.

En particulier, on peut citer la glycérine à titre d'humidifiant. On peut encore citer à titre d'huiles synthétiques les cyclométhicones, en particulier la pentacyclométhicone.

La composition cosmétique selon l'invention peut être utilisée dans un mode rincé ou dans un mode non rincé.

Lorsque la composition cosmétique est destinée à être appliquée sur les cheveux, elle peut se présenter sous forme d'une lotion, d'un spray, d'une mousse, d'une cire, d'un gel, d'une pâte, d'un shampooing ou d'un après shampooing, ou d'un soin d'une composition de permanente, d'un produit de lissage permanent ou non, ou d'un produit de coloration directe ou d'oxydation, d'un produit de défrisage, d'un produit de décoloration.

Lorsque la composition est destinée à être appliquée sur la peau, elle peut se présenter sous la forme d'une composition de soin, par exemple une composition nettoyante ou de maquillage, telle qu'une lotion, un gel, une mousse, une crème solaire, une crème de soin, un masque, un gel nettoyant, une huile, un rouge à lèvre, un eye-liner, un mascara, une poudre, un fard.

La composition cosmétique selon l'invention peut être sous forme d'émulsion huile-eau ou eau-dans-huile.

Lorsque la composition cosmétique selon l'invention est sous forme d'émulsion, elle contient généralement au moins un agent tensio-actif non-ionique, anionique, cationique ou amphotère.

Les émulsionnants sont choisis de manière appropriée suivant l'émulsion à obtenir : émulsion eau-dans-l'huile (E/H) ou huile-dans-eau (H/E).

Pour les émulsions huile-dans-eau (H/E), on peut citer par exemple les émulsionnants suivants:
* comme émulsionnants amphotères, les N-acyl-aminoacides tels que les N-alkylaminoacétates et le cocoamphodiacétate disodique et les oxydes d'amines tels que l'oxyde de stéaramine;
* comme émulsionnants anioniques, les acylglutamates tels que le "disodium hydrogenated tallow glutamate" (Amisoft HS-21® commercialisé par la société AJINOMOTO) ; les acides carboxyliques et leurs sels tels que le stéarate de sodium ; les esters phosphoriques et leurs sels tels que le "DEA oleth-10 phosphate" ; les sulfosuccinates tels que le "Disodium PEG-5 citrate lauryl sulfosuccinate" et le "Disodium ricinoleamido MEA sulfosuccinate" ;
* comme émulsionnants cationiques, les alkyl-imidazolidinium tels que l'éthosulfate d'isostéaryl-éthylimidonium ; les sels d'ammonium tels que le chlorure de N,N,N-triméthyl-1-docosanaminium (Behentrimonium chloride);
* comme émulsionnants non ioniques, les esters et éthers d'oses tels que le stéarate de sucrose, le cocoate de sucrose, et le mélange de stéarate de sorbitan et de cocoate de sucrose commercialisé par la société ICI sous la dénomination Arlatone 2121® ; les esters de polyol, notamment de glycérol ou de sorbitol, tels que le stéarate de glycéryle, le stéarate de polyglycéryl-2, le stéarate de sorbitan ; les éthers de glycérol ; les éthers oxyéthylénés et/ou oxypropylénés, tels que l'éther oxyéthyléné, oxypropyléné de l'alcool laurique à 25 groupes oxyéthylénés et 25 groupes oxypropylénés (nom CTFA "PPG-25 laureth-25") et l'éther oxyéthyléné du mélange d'alcools gras en C₁₂-C₁₅ comportant 7 groupes oxyéthylénés (nom CTFA " C₁₂-C₁₅ Pareth-7") ; les polymères d'éthylène glycol, tels que le PEG-100, et leurs mélanges.

On peut utiliser un ou plusieurs de ces émulsionnants.

Pour les émulsions eau-dans-huile (E/H), on peut citer comme exemple d'émulsionnants, les esters gras de polyol, notamment de glycérol ou de sorbitol, et notamment les esters isostéariques, oléiques et ricinoléiques de polyol, tels que le mélange de petrolatum, d'oléate de polyglycéryl-3, d'isostéarate de glycéryle, l'huile de ricin hydrogénée et d'ozokérite, vendu sous la dénomination Protegin W® par la société GOLDSCHMIDT, l'isostéarate de sorbitan, le di-isostéarate de polyglycéryle, le sesqui-isostéarate de polyglycéryle-2 ; les esters et éthers d'oses tels que le "Methyl glucose dioléate" ; les esters gras tels que le lanolate de magnésium ; les diméthicone copolyols et alkyl-diméthicone copolyols tels que le laurylméthicone copolyol vendu sous la dénomination Dow Corning 5200 Formulation Aid par la société DOW CORNING, le Cétyl diméthicone copolyol vendu sous la dénomination Abil EM 90® par la société GOLDSCHMIDT, et le diméthicone copolyol vendu sous la dénomination KF-6015 par la société SHINETSU et leurs mélanges.

La composition cosmétique selon l'invention peut être conditionnée sous forme de composition aérosol. Dans ce cas, elle contient au moins un gaz propulseur.

La composition selon l'invention peut être utilisée pour la mise en forme des cheveux.

L'invention a encore pour objet l'utilisation dans une composition cosmétique d'un composé à structure cage tel que défini précédemment pour améliorer l'effet tenseur de la composition le comprenant.

L'invention a encore pour objet l'utilisation dans une composition cosmétique comprenant un ou plusieurs polymères, d'un composé à structure cage tel que défini précédemment pour améliorer la résistance mécanique du ou desdits polymères.

L'invention a enfin pour objet l'utilisation dans une composition cosmétique de mascara d'un composé à structure cage tel que défini précédemment pour améliorer le recourbement des cils.

La présente invention est illustrée par les exemples suivants.

### Exemple 1 : Lotion coiffante en spray

| | |
|---|---|
| Composé alumoxane à structure cage à structure cubique fermée substitué par des groupements carboxyliques | 1,0% |
| Copolymère acrylate de butyle/acide acrylique/acide méthacrylique | 3,0% |
| Ethanol | 20,0% |
| Eau | 76,0% |

Le composé à structure cage utilisé est décrit dans la publication suivante : Callender, R.L., C.J.Harlan, N.M.Shapiro, C.D.Jones, D.B.Macqueen, D.L.Callahan, M.R.Wiesner, R.Cook and A.R Barron, *Chemistry of Materials,* 9(11), (1997), 2418-2433.

### Exemple 2 : Lotion coiffante en spray

| | |
|---|---|
| Composé à structure cage à base de fer substitué par des groupements carboxyliques | 1,0% |
| Copolymère acrylate de butyle/acide acrylique/acide méthacrylique | 3,0% |
| Ethanol | 20,0% |
| Eau | 76,0% |

Le composé à structure cage utilisé est décrit dans la publication suivante : J.Rose, M.Cortalezzi-Fidalgo, S.Moustier, C.Magnetto, C.Jones, A.Barron, M.Wiesner, JY Bottero, *Chemistry of Materials,* 14(2), (2002), 621-628.

### Exemple 3 : Fluide de soin

### 1. Phase aqueuse

| | |
|---|---|
| Composé à structure cage à base d'aluminium substitué par des groupements carboxyliques³ | 1,0% |
| chlorure de sodium | 2,50% |
| glycérine | 7,00% |
| Eau | 70,00% |

Le composé à structure cage utilisé est décrit dans la publication suivante : 1 Callender, R.L., C.J.Harlan, N.M.Shapiro, C.D.Jones, D.B.Macqueen, D.L.Callahan, M.R.Wiesner, R.Cook and A.R Barron, *Chemistry of Materials,* **9(11)** , (1997), 2418-2433.

### 2. Phase huileuse

| | |
|---|---|
| diméthicone copolyol (commercialisé sous la référérence KF-6015 par la société SHINETSU) | 1,75% |
| pentacyclométhicone | 17,75% |

## Revendications

1. Composition cosmétique comprenant dans un milieu cosmétiquement acceptable au moins un composé ayant une structure cage ouverte ou fermée, **caractérisée en ce que** le ou lesdits composés comprennent au moins 4 atomes d'un élément choisi parmi les éléments des colonnes 3 à 13 de la classification périodique, et au moins 4 atomes d'oxygène, lesdits atomes dudit élément étant liés uniquement à des atomes d'oxygène et à un ou plusieurs substituants identiques ou différents.

2. Composition selon la revendication 1 **caractérisée en ce que** le ou lesdits composés comprennent entre 4 et 20, de préférence entre 4 et 12, mieux 6, 7, 8, 9, 10 ou 12 atomes dudit élément.

3. Composition selon la revendication 1 ou 2 **caractérisée en ce que** le ou lesdits composés ont une structure cage fermée.

4. Composition selon l'une quelconque des revendications précédentes **caractérisée en ce que** la structure cage du ou desdits composés forme un feuillet.

5. Composition selon l'une quelconque des revendications 1 à 3 **caractérisée en ce que** la structure cage du ou desdits composés forme un groupement.

6. Composition selon l'une quelconque des revendications précédentes **caractérisée en ce que** l'élément est choisi parmi les éléments des colonnes 8 à 13, de préférence le fer, l'aluminium et le gallium.

7. Composition selon l'une quelconque des revendications précédentes **caractérisés en ce que** les substituants sont choisis parmi l'hydrogène ou parmi les groupes hydroxyle, alkyle, alkylène, alcényle, aryle, acyle, alcoxy, de préférence méthoxy, hydride, ester, carboxyle, acrylate, alkyl acrylate, alcool, aldéhyde, amine, alkylamine, silanol, les groupes contenant une ou plusieurs fonctions amines, les groupes siloxane, les groupes contenant un ou plusieurs groupes siloxane, les groupes silicone ou les groupes contenant un ou plusieurs groupes silicones, les groupe silane ou les groupes contenant un ou plusieurs groupes silanes, les groupes contenant un ou plusieurs atomes de fluor, de soufre ou de phosphore, les groupes SO₂, CO₂X, SO₃X ou X est un atome d'hydrogène, un méthyl ou un éthyl, les groupes alpha-oléphines, époxyde, azo, diazo, halogène, les groupes cycliques pouvant donner lieu à une isomérisation par ouverture de cycle, la silice moléculaire, les groupe nitrile, thiol, et les polymères, de préférence les polyacrylates, les résines époxy, les résines phénol-formaldéhyde, les polyamides, les polyesters, les polyimides, les polycarbonates, les polyuréthannes et les polymères quinone amine.

8. Composition selon la revendication 7 **caractérisée en ce que** les substituants sont choisis parmi les groupes hydroxyle, alkyle, phénol, amine quaternaire, haloalkyle, méthacrylate, halosilane, styrène, norbornényle et terbutyle.

9. Composition selon l'une quelconque des revendications précédentes **caractérisée en ce que** le ou les composés ayant une structure cage sont choisis parmi le [(^{t}Bu)M(µ₃-O)]₆, le [(^{t}Bu)M(µ₃-O)]₇, le [(^{t}Bu)Al(µ₃₋0)]₈, le [(^{t}Bu)M(µ₃-O)]₉, le [(^{t}Bu)M(µ₃-O]₁₀, le [(^{t}Bu)M(µ₃-O]₁₂, où M représente AI ou Ga.

10. Composition selon l'une quelconque des revendications précédentes **caractérisée en ce que** elle comprend de 0,00001 à 20%, de préférence de 0,0001 à 10%, mieux de 0,001 à 5%, en poids de composé(s) ayant une structure cage.

11. Composition selon l'une quelconque des revendications précédentes **caractérisée en ce que** elle comprend un solvant choisi parmi l'eau, les alcools inférieurs en C₁-C₄, de préférence l'éthanol et l'isopropanol, les cétones en C₃-C₆, de préférence l'acétone et la méthyléthylcétone, les esters en C₃-C₆, de préférence l'acétate d'éthyle et l'acétate de butyle, les éthers en C₂-C₆, de préférence le diéthoxyéthane et le diméthoxyéthane, et les alcanes en C₆-C₁₀.

12. Composition selon l'une quelconque des revendications précédentes **caractérisée en ce qu'**elle contient au moins un polymère fixant.

13. Composition selon l'une quelconque des revendications précédentes **caractérisée en ce qu'**elle se présente sous la forme d'une émulsion huile-dans-eau ou eau-dans-huile.

14. Utilisation d'une composition telle que définie à l'une quelconque des revendications précédentes pour la mise en forme des cheveux.

15. Utilisation dans une composition cosmétique d'un composé à structure cage tel que défini à l'une quelconque des revendications 1 à 9 pour améliorer l'effet tenseur de la composition le comprenant.

16. Utilisation dans une composition cosmétique comprenant un ou plusieurs polymères, d'un composé à structure cage tel que défini à l'une quelconque des revendications 1 à 9 pour améliorer la résistance mécanique dudit ou desdits polymères.

17. Utilisation dans une composition cosmétique de mascara d'un composé à structure cage tel que défini à l'une quelconque des revendications 1 à 9 pour améliorer le recourbement des cils.
